# EUROPEAN PATENT APPLICATION

(11) **EP 4 552 507 A1**
(43) Date of publication of application: **14.05.2025**
(21) Application number: 23209201.5
(22) Date of filing: 10.11.2023
(51) Int. Cl.: A23K 10/16, A23K 20/147, A23L 33/195, A23K 10/12, A23L 33/135, A23J 3/20, C12N 1/20

(54) **METHYLOTROPH BACTERIA AS FOOD AND FEED INGREDIENTS**

(71) Applicant: Acies Bio d.o.o., 1000 Ljubljana (SI)
(72) Inventor: VIRANT, David, Ljubljana (SI); KOSEC, Gregor, Ljubljana (SI); KRUIS, Aleksander Johannes, Ljubljana (SI); JIA, Wanqing, Bennekom (NL); CLAASSENS, Nico, Renkum (NL); POUVREAU, Laurice, Bennekom (NL); KEPPLER, Julia, Wageningen (NL)
(74) Representative: Zacco GmbH

(57) **Abstract**

The present invention provides the use of a protein composition comprising Methylotroph bacterial biomass or at least one fraction thereof for supplementing or substituting meat proteins in food or feed products. Also provided are food and feed ingredients, food and feed products and methods of making same.

## Description

### Technical field of the invention

The present invention relates to the the field of biotechnology, microbiology, food and feed products. In particular the present invetion relates to Methyltroph bacterial biomass and fractions thereof and their use as food and feed ingredients.

### Background

Due to the ever increasing global population there is an increasing demand for food and feed. Also, the is an increasing global demand for high-quality protein and the growing awareness among consumers that the disproportionate consumption of meat proteins is not sustainable and has a negative impact on animal welfare. Thus, there is a need and growing interest in the search for alternative protein sources.

Plant proteins are promising, but often have deficiencies in technical functionality and sensory characteristics that limit their application range for imitating meat and dairy products.

Another route is so-called single cell protein (SCP) or cellular protein, where fungi (Quorn), yeast, bacteria or microalgae are investigated for their protein yield and composition as food ingredient. Growing bacteria on one-carbon compounds, such as methane, has been tested for more than 50 years as an alternative protein source to plants and meat. Among the obstacles have been e.g. the rather high contents of nucleic acids, and obtaining an acceptable flavor and odor, texturizing the product for food applications etc.

US3845222 discloses a texturizing process for single cell protein from bacteria or yeasts.

There is a need for sustainable and functional alternative protein sources to animal proteins. Also, there is a need for such alternative protein sources which have a suitable amino acid profile for human and animal nutrition, and which have acceptable functional properties when used in foods. In particular there is a need for a protein source being amenable for effective production, having acceptable color and smell, and which possesses physicochemical and rheological properties which makes it suitable as a component in foods without excessive modifications.

### Summary of the invention

The present inventors have characterised bacterial biomass from the Methylotroph *Methylobacillus* as well fractions thereof and surprisingly found that the biomass and the fractions possess some excellent properties for being used for supplementing or substituting meat proteins in foods and feed. In particular the biomass and the fractions have a pleasant color and smell, as well as good structural and rheological properties.

In a first aspect the present invention provides the use of a protein composition comprising Methylotroph bacterial biomass or at least one fraction thereof for supplementing or substituting meat proteins in food or feed products.

In an embodiment the Methylotroph bacterial biomass is *Methylobacillus.*

In a second aspect the present invention provides a food or feed ingredient comprising Methylotroph bacterial biomass or at least one fraction thereof.

In a third aspect the present invention provides a food or feed product comprising Methylotroph bacterial biomass or at least one fraction thereof.

In a fourth aspect the present invention provides a method for making a food or feed ingredient comprising:
- growing a Methylotroph bacterium to a density allowing the recovery of biomass,
- optionally disrupting said recovered biomass to make a disrupted biomass (DB),
- optionally subjecting said disrupted biomass to centrifugation to produce a pellet-insoluble fraction (IF) and a soluble fraction (SF),
- optionally subjecting the soluble fraction (SF) to acid precipitation followed by neutralization to produce the acid insoluble fraction (AIF),
- reducing the water contents in said biomass, DB, IF, SF or AIF to less than 5.0 %w/w,
to form said food or feed ingredient.

The present invention is further characterized by the following items:
1. Use of a protein composition comprising Methylotroph bacterial biomass or at least one fraction thereof for supplementing or substituting proteins in food or feed products.
2. The use according to item 1, wherein said protein composition is used for supplementing or substituting meat proteins in food or feed products.
3. The use according to item 1 or 2, wherein said protein composition is used for supplementing or substituting plant proteins in food or feed products.
4. The use according to any one of items 1-3, wherein said protein composition comprises Methylotroph bacterial biomass.
5. The use according to any one of items 1-4, wherein said protein composition comprises at least one fraction of Methylotroph bacterial biomass.
6. The use according to any one of items 1-5, wherein said at least one fraction of Methylotroph bacterial biomass comprises disrupted biomass (DB).
7. The use according to any one of items 1-6, wherein said at least one fraction of Methylotroph bacterial biomass comprises the pellet-insoluble fraction (IF) as obtainable as the precipitate from centrifugation of disrupted biomass.
8. The use according to any one of items 1-7, wherein said at least one fraction of Methylotroph bacterial biomass comprises the soluble fraction (SF) as obtainable as the supernatant from centrifugation of disrupted biomass.
9. The use according to any one of items 1-8, wherein said at least one fraction of Methylotroph bacterial biomass comprises the acid insoluble fraction (AIF) as obtainable as the insoluble fraction following neutralization of the precipitate from the centrifugation of disrupted biomass.
10. The use according to any one of items 1-9, wherein said methylotroph bacterial biomass is from the genus *Methylobacillus.*
11. The use according to item 10, wherein said Methylotroph bacterial biomass is from *Methylobacillus* which does not express exopolysaccharides.
12. The use according to item 10 or 11, wherein said Methylotroph bacterial biomass is from *Methylobacillus* which has at least one EPS gene cluster inactivated.
13. The use according to any one of items 10-12, wherein said Methylotroph bacterial biomass is from *Methylobacillus* which has both EPS gene clusters inactivated.
14. The use according to item 12 or 13, wherein said at least one EPS gene cluster or said both EPS gene clusters are entirely or partly deleted (absent).
15. The use according to any one of items 10-14, wherein said Methylotroph bacterial biomass is from *Methylobacillus* which has a decreased, inactivated or deleted polyphosphate kinase (PPK).
16. The use according to any one of items 10-15, wherein said Methylotroph bacterial biomass is from *Methylobacillus* which has a decreased, inactivated or deleted Acyl-homoserine-lactone (AHL) synthase.
17. The use according to any one of items 1-16, wherein said Methylotroph bacterial biomass is from a bacterium selected from the group consisting of *Methylobacillus flagellatus, Methylobacillus glycogenes, Methylobacillus pratensis* and *Methylobacillus rhizosphaerae.*
18. The use according to item 17, wherein said Methylotroph bacterial biomass is *Methylobacillus flagellatus* biomass.
19. The use according to any one of items 1-18, which comprises the addition of said Methylotroph bacterial biomass or a fraction thereof such that it constitutes from 10%w/w(DM) to 75%w/w(DM) of the food or feed product, from 10%w/w(DM) to 50%w/w(DM) of the food or feed product or from 10%w/w(DM) to 35%w/w(DM) of the food or feed product.
20. The use according to any one of items 1-19, which comprises the addition of said Methylotroph bacterial biomass or a fraction thereof such that its protein contents in said food or feed product is in the range from 10%w/w to 100%w/w of the total protein contents in said food or feed product, in the range from 10%w/w to 75%w/w of the total protein contents in said food or feed product or from 10%w/w to 50%w/w of the total protein contents in said food product.
21. The use according to any one of items 1-20, wherein said food or feed product is a solid product.
22. The use according to any one of items 1-21, wherein said food or feed product is a meat like product, such as a meat substitute or meat supplemented with proteins.
23. The use according to any one of items 1-22, wherein said food or feed product is a food product.
24. The use according to any one of items 1-23, wherein said composition comprises Methylotroph bacterial biomass or at least one fraction thereof for substituting meat proteins in food or feed products.
25. The use according to any one of items 1-22, wherein said food or feed product is a feed product.
26. The use according to item 25, wherein said feed product is an animal feed product.
27. The use according to item 25 or 26, wherein said feed product is an animal feed product for farm animals or for pets.
28. The use according to any one of items 1-27, wherein said composition comprises Methylotroph bacterial biomass or at least one fraction thereof for supplementing meat proteins in food or feed products.
29. Food or feed ingredient comprising Methylotroph bacterial biomass or at least one fraction thereof.
30. The food or feed ingredient according to item 29, which comprises Methylotroph bacterial biomass.
31. The food or feed ingredient according to item 29 or 30, which comprises at least one fraction of Methylotroph bacterial biomass.
32. The food or feed ingredient according to any one of items 29-31, wherein said at least one fraction of Methylotroph bacterial biomass comprises disrupted biomass (DB).
33. The food or feed ingredient according to any one of items 29-32, wherein said at least one fraction of Methylotroph bacterial biomass comprises the pellet-insoluble fraction (IF) as obtainable as the precipitate from centrifugation of disrupted biomass.
34. The food or feed ingredient according to any one of items 29-33, wherein said at least one fraction of Methylotroph bacterial biomass comprises the soluble fraction (SF) as obtainable as the supernatant from centrifugation of disrupted biomass.
35. The food or feed ingredient according to any one of items 29-34, wherein said at least one fraction of Methylotroph bacterial biomass comprises the acid insoluble fraction (AIF) as obtainable as the insoluble fraction following neutralization of the precipitate from the centrifugation of disrupted biomass.
36. The food or feed ingredient according to any one of items 29-35, wherein said Methylotroph bacterial biomass is from the genus *Methylobacillus.*
37. The food or feed ingredient according to item 36, wherein said Methylotroph bacterial biomass is from *Methylobacillus* which does not express exopolysaccharides.
38. The food or feed ingredient according to item 36 or 37, wherein said Methylotroph bacterial biomass is from *Methylobacillus* which has at least one EPS gene cluster inactivated.
39. The food or feed ingredient according to any one of items 36-38, wherein said Methylotroph bacterial biomass is from *Methylobacillus* which has both EPS gene clusters inactivated.
40. The food or feed ingredient according to items 38 or 39, wherein said at least one EPS gene cluster or said both EPS gene clusters are entirely or partly deleted (absent).
41. The food or feed ingredient according to any one of items 29-40, wherein said Methylotroph bacterial biomass is from *Methylobacillus* which has a decreased, inactivated or deleted polyphosphate kinase (PPK).
42. The food or feed ingredient according to any one of items 29-41, wherein said Methylotroph bacterial biomass is from *Methylobacillus* which has a decreased, inactivated or deleted Acyl-homoserine-lactone (AHL) synthase.
43. The food or feed ingredient according to any one of items 29-42, wherein said Methylotroph bacterial biomass is from a bacterium selected from the group consisting of *Methylobacillus flagellatus, Methylobacillus glycogenes, Methylobacillus pratensis* and *Methylobacillus rhizosphaerae.*
44. The food or feed ingredient according to item 43, wherein said Methylotroph bacterial biomass is *Methylobacillus flagellatus* biomass.
45. The food or feed ingredient according to any one of items 29-44, wherein said food ingredient is a solid composition.
46. The food or feed ingredient according to item 45, wherein said food ingredient has a water contents of less than 5.0 %w/w.
47. The food or feed ingredient according to any of items 29-46, which is a food ingredient.
48. The food or feed ingredient according to any one of items 29-46, which is a feed ingredient.
49. Food or feed product comprising Methylotroph bacterial biomass or at least one fraction thereof.
50. The food or feed product according to item 49, wherein said protein composition comprises Methylotroph bacterial biomass.
51. The food or feed product according to item 49 or 50, wherein said protein composition comprises at least one fraction of Methylotroph bacterial biomass.
52. The food or feed product according to item 49 or 50, wherein said at least one fraction of Methylotroph bacterial biomass comprises disrupted biomass (DB).
53. The food or feed product according to any one of items 49-52, wherein said at least one fraction of Methylotroph bacterial biomass comprises the pellet-insoluble fraction (IF) as obtainable as the precipitate from centrifugation of disrupted biomass.
54. The food or feed product according to any one of items 49-53, wherein said at least one fraction of Methylotroph bacterial biomass comprises the soluble fraction (SF) as obtainable as the supernatant from centrifugation of disrupted biomass.
55. The food or feed product according to any one of items 49-54, wherein said at least one fraction of Methylotroph bacterial biomass comprises the acid insoluble fraction (AIF) as obtainable as the insoluble fraction following neutralization of the precipitate from the centrifugation of disrupted biomass.
56. The food or feed product according to any one of items 49-55, wherein said Methylotroph bacterial biomass is from the genus *Methylobacillus.*
57. The food or feed product according to item 56, wherein said Methylotroph bacterial biomass is from a *Methylobacillus* strain which does not express exopolysaccharides.
58. The food or feed product according to item 56 or 57, wherein said Methylotroph bacterial biomass is from *Methylobacillus* which has at least one EPS gene cluster inactivated.
59. The food or feed product according to any one of items 56-58, wherein said Methylotroph bacterial biomass is from *Methylobacillus* which has both EPS gene clusters inactivated.
60. The food or feed product according to any one of items 58-59, wherein said at least one EPS gene cluster or said both EPS gene clusters are entirely or partly deleted (absent).
61. The food or feed product according to any one of items 49-60, wherein said Methylotroph bacterial biomass is from *Methylobacillus* which has a decreased, inactivated or deleted polyphosphate kinase (PPK).
62. The food or feed product according to any one of items 49-61, wherein said Methylotroph bacterial biomass is from *Methylobacillus* which has a decreased, inactivated or deleted Acyl-homoserine-lactone (AHL) synthase.
63. The food or feed product according to any one of items 49-62, wherein said Methylotroph bacterial biomass is from a bacterium selected from the group consisting of *Methylobacillus flagellatus, Methylobacillus glycogenes, Methylobacillus pratensis* and *Methylobacillus rhizosphaerae.*
64. The food or feed product according to item 63, wherein said Methylotroph bacterial biomass is *Methylobacillus flagellatus* biomass.
65. The food or feed product according to any one of items 49-64, which comprises from 10 %w/w(DM) to 75 %w/w(DM) of said Methylotroph bacterial biomass or a fraction thereof, from 10 %w/w(DM) to 50 %w/w(DM) of said Methylotroph bacterial biomass or a fraction thereof or from 10 %w/w(DM) to 35 %w/w(DM) of said Methylotroph bacterial biomass or a fraction thereof.
66. The food or feed product according to any one of items 49-65, which comprises said Methylotroph bacterial biomass or a fraction thereof in an amount such that its protein contents from said Methylotroph biomass in said food product is in the range from 10 %w/w to 100 %w/w of the total protein contents in said food or feed product, in the range from 10 %w/w to 75 %w/w of the total protein contents in said food or feed product or from 10 %w/w to 50 %w/w of the total protein contents in said food or feed product.
67. The food or feed product according to any one of items 49-66, wherein said food or feed product is a solid product.
68. The food or feed product according to any one of items 49-67, which is a meat like product, such as a meat substitute or meat supplemented with proteins.
69. The food or feed product according to any of items 49-68, which is a feed product.
70. The food or feed product according to any of items 49-69, which is an animal feed product for farm animals or for pets.
71. The food or feed product according to any of items 49-68, which is a food product.
72. The food product according to any one of items 49-68 and 71, which is a burger patty.
73. A method for making a food or feed ingredient comprising:
   - growing a Methylotroph bacterium to a density allowing the recovery of biomass,
   - optionally disrupting said recovered biomass to make a disrupted biomass (DB),
   - optionally subjecting said disrupted biomass to centrifugation to produce a pellet-insoluble fraction (IF) and a soluble fraction (SF),
   - optionally subjecting the soluble fraction (SF) to acid precipitation followed by neutralization to produce the acid insoluble fraction (AIF),
   - reducing the water contents in said biomass, DB, IF, SF or AIF to less than 5.0 %w/w,
   to form said food or feed ingredient.
74. The method according to item 73, wherein said Methylotroph bacterium is *Methylobacillus,* such as *Methylobacillus flagellatus, Methylobacillus glycogenes, Methylobacillus pratensis* or *Methylobacillus rhizosphaerae*
75. The method according to item 73 or 74, wherein the reduction of the water contents of the biomass or fraction thereof is done by spray drying or by freeze drying.
76. The method according to any one of items 73-75, wherein said food or feed ingredient is a food ingredient.
77. The method according to any one of items 73-75, wherein said food or feed ingredient is a feed ingredient.

### Description of the figures

Figure 1. Bioprocess diagram of the *Methylobacillus OCB420* biomass production process.
Figure 2: Schematic of the procedure used to obtain different sample fractions (UB, DB, IF, SF, AIF)
Figure 3: Confocal microscope images of untreated (left) and freeze dried (right) biomass stained with Rhodamine-B (gray) and Bodipy (white).
Figure 4: protein content of commerical food/feed protein sources.
Figure 5: Comparison of amino acid content of commercial food/feed protein sources fish meal, soy protein concentrate, pea protein isolate. Values shown as grams of amino acid per 100g dry weight.
Figure 6: Protein molecular weight distribution by SDS-PAGE without reducing agent (A) and with reducing agent (B) for the freeze-dried samples of UB, DB, SF, IF and AIF.
Figure 7: A) FTIR spectra of the sample fractions UB, DB, SF, IF and AIF and B) second derivative of the amide I band of the same samples.
Figure 8: A) Heat flow of the sample fractions UB, DB, SF, AIF and IF by heating from 20 to 130 °C for the first run, and B) heat flow of the sample fractions for the second run.
Figure 9: A) Zeta potential (A), conductivity (B), water holding capacity (g water/g dry pellet) (C) and Color intensity (D) of UB, DB, SF, IF and AIF. The color intensity was indicated as L* (perceptual lightness), a* (-a* = green and + a* = red) and b* (-b* = blue and + b* = yellow). The color was measured with ColorFlexEZ (HunterLab).
Figure 10. Structuring properties of freeze-dried UB (A1, A2 and A3), AIF (B1, B2 and B3) and SPC (C1 and C2) (dry matter of 40%), corresponding to a protein concentration of approximately 28% in the shear cell.

### Description

Unless specifically defined herein, all technical and scientific terms used have the same meaning as commonly understood by a skilled artisan in the fields of biochemistry, genetics, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, fermentation technology, recombinant DNA, and food science, which are within the skill of the art. Such techniques are explained fully in the literature.

In a first aspect the present invention provides the use of a protein composition comprising Methylotroph bacterial biomass or at least one fraction thereof for substituting meat proteins in food or feed products.

Methylotroph bacterial biomass may be obtained by the cultivation of methylotrophic bacteria. In one embodiment the Methylotroph bacterial biomass is from the genus *Methylobacillus.* In a further embodiment the Methylotroph bacterial biomass is from *Methylobacillus* which does not express exopolysaccharides. In a further embodiment the Methylotroph bacterial biomass is from *Methylobacillus* which has at least one EPS gene cluster inactivated. In yet another embodiment the Methylotroph bacterial biomass is from *Methylobacillus* which has both EPS gene clusters inactivated. In a further embodiment the at least one EPS gene cluster or said both EPS gene clusters are entirely or partly deleted (absent). In another embodiment the Methylotroph bacterial biomass is from *Methylobacillus* which has a decreased, inactivated or deleted polyphosphate kinase (PPK). In another embodiment the Methylotroph bacterial biomass is from *Methylobacillus* which has a decreased, inactivated or deleted Acyl-homoserine-lactone (AHL) synthase.

In a further embodiment the Methylotroph bacterial biomass is from a bacterium selected from the group consisting of *Methylobacillus flagellatus, Methylobacillus glycogenes, Methylobacillus pratensis, Methylobacillus rhizosphaerae.* In a further embodiment the Methylotroph bacterial biomass is *Methylobacillus flagellatus* biomass.

In an embodiment the protein composition comprises Methylotroph bacterial biomass. In another embodiment the protein composition is Methylotroph bacterial biomass In another embodiment the protein composition comprises at least one fraction of Methylotroph bacterial biomass.

In another embodiment the at least one fraction of Methylotroph bacterial biomass comprises disrupted biomass (DB). In another embodiment the at least one fraction of Methylotroph bacterial biomass is disrupted biomass (DB).

In another embodiment the at least one fraction of Methylotroph bacterial biomass comprises the pellet-insoluble fraction (IF) as obtainable as the precipitate from centrifugation of disrupted biomass. In another embodiment the at least one fraction of Methylotroph bacterial biomass is the pellet-insoluble fraction (IF) as obtainable as the precipitate from centrifugation of disrupted biomass.

In another embodiment the at least one fraction of Methylotroph bacterial biomass comprises the soluble fraction (SF) as obtainable as the supernatant from centrifugation of disrupted biomass. In another embodiment the at least one fraction of Methylotroph bacterial biomass is the soluble fraction (SF) as obtainable as the supernatant from centrifugation of disrupted biomass.

In another embodiment the at least one fraction of Methylotroph bacterial biomass comprises the acid insoluble fraction (AIF) as obtainable as the insoluble fraction following neutralization of the precipitate from the centrifugation of disrupted biomass. In another embodiment the at least one fraction of Methylotroph bacterial biomass is the acid insoluble fraction (AIF) as obtainable as the insoluble fraction following neutralization of the precipitate from the centrifugation of disrupted biomass.

Varying amounts of said Methylotroph bacterial biomass or a fraction thereof may be added to the food or feed product dependent on the particular application, and the desired protein contents, rheological considerations etc.

In an embodiment the use comprises the addition of said Methylotroph bacterial biomass or a fraction thereof such that it constitutes from 10%w/w(DM) to 75%w/w(DM) of the food or feed product, from 10%w/w(DM) to 50%w/w(DM) of the food or feed product or from 10%w/w(DM) to 35%w/w(DM) of the food or feed product.

In a further embodiment the use comprises the addition of said Methylotroph bacterial biomass or a fraction thereof such that its protein contents in said food or feed product is in the range from 10%w/w to 100%w/w of the total protein contents in said food or feed product, in the range from 10%w/w to 75%w/w of the total protein contents in said food or feed product or from 10%w/w to 50%w/w of the total protein contents in said food product.

The food or feed product may take any form, e.g. being a solid product, a liquid or a gel product. In an embodiment the food or feed product is a solid product.

In another embodiment the food or feed product is a meat like product, such as a meat substitute.

In another embodiment the food or feed product is a food product.

In another embodiment the said food or feed product is a feed product.

In another embodiment the feed product is an animal feed product.

In another embodiment the feed product is an animal feed product for farm animals or for pets.

In another embodiment the feed ingredient comprises Methylotroph bacterial biomass or at least one fraction thereof.

In another embodiment the food or feed ingredient comprises Methylotroph bacterial biomass.

In another embodiment the food or feed ingredient comprises at least one fraction of Methylotroph bacterial biomass.

In another embodiment the food or feed ingredient comprises at least one fraction ofMethylotroph bacterial biomass comprising disrupted biomass (DB). In another embodiment the food or feed ingredient comprises at least one fraction ofMmethylotroph bacterial biomass which is disrupted biomass (DB).

In another embodiment the food or feed ingredient comprises at least one fraction of Methylotroph bacterial biomass comprising the pellet-insoluble fraction (IF) as obtainable as the precipitate from centrifugation of disrupted biomass. In another embodiment the food or feed ingredient comprises at least one fraction of Methylotroph bacterial biomass which is the pellet-insoluble fraction (IF) as obtainable as the precipitate from centrifugation of disrupted biomass.

In another embodiment the food or feed ingredient comprises at least one fraction of Methylotroph bacterial biomass comprising the soluble fraction (SF) as obtainable as the supernatant from centrifugation of disrupted biomass. In another embodiment the food or feed ingredient comprises at least one fraction of Methylotroph bacterial biomass which is the soluble fraction (SF) as obtainable as the supernatant from centrifugation of disrupted biomass.

In another embodiment the food or feed ingredient comprises at least one fraction of Methylotroph bacterial biomass comprising the acid insoluble fraction (AIF) as obtainable as the insoluble fraction following neutralization of the precipitate from the centrifugation of disrupted biomass. In another embodiment the food or feed ingredient comprises at least one fraction of Methylotroph bacterial biomass which is the acid insoluble fraction (AIF) as obtainable as the insoluble fraction following neutralization of the precipitate from the centrifugation of disrupted biomass.

In another embodiment the food or feed ingredient comprises Methylotroph bacterial biomass from the genus *Methylobacillus.*

In another embodiment the food or feed ingredient comprises Methylotroph bacterial biomass from *Methylobacillus*which does not express exopolysaccharides.

In another embodiment the food or feed ingredient comprises Methylotroph bacterial biomass from *Methylobacillus* which has at least one EPS gene cluster inactivated.

In another embodiment the food or feed ingredient comprises Methylotroph bacterial biomass from *Methylobacillus*which has both EPS gene clusters inactivated.

In another embodiment the food or feed ingredient comprises Methylotroph bacterial biomass wherein said at least one EPS gene cluster or said both EPS gene clusters are entirely or partly deleted (absent).

In another embodiment the food or feed ingredient comprises Methylotroph bacterial biomass which is from *Methylobacillus* which has a decreased, inactivated or deleted polyphosphate kinase (PPK).

In another embodiment the food or feed ingredient comprises Methylotroph bacterial biomass which is from *Methylobacillus* which has a decreased, inactivated or deleted Acyl-homoserine-lactone (AHL) synthase.

*Methyllobacillus* strains having a decreased, inactivated or deleted polyphosphate kinase (PPK) and/or a decreased, inactivated or deleted Acyl-homoserine-lactone (AHL) synthase are disclosed in e.g. WO2023/139255.

In another embodiment the food or feed ingredient comprises Methylotroph bacterial biomass from a bacterium selected from the group consisting of *Methylobacillus flagellatus, Methylobacillus glycogenes, Methylobacillus pratensis, Methylobacillus rhizosphaerae.*

In another embodiment the food or feed ingredient comprises Methylotroph bacterial biomass which is *Methylobacillus flagellatus* biomass.

In another embodiment the food or feed ingredient is a solid composition.

In another embodiment the food or feed ingredient has a water contents of less than 5.0 %w/w.

In another embodiment the food or feed ingredient is a food ingredient.

In another embodiment the food or feed ingredient is a feed ingredient.

In a further aspect is provided a food or feed product comprising Methylotroph bacterial biomass or at least one fraction thereof for substituting meat proteins in food products.

In another embodiment the food or feed product comprises Methylotroph bacterial biomass.

In another embodiment the food or feed product comprises at least one fraction of Methylotroph bacterial biomass.

In another embodiment the food or feed product comprises at least one fraction of Methylotroph bacterial biomass comprising disrupted biomass (DB).

In another embodiment the food or feed product comprising Methylotroph bacterial biomass comprising the pellet-insoluble fraction (IF) as obtainable as the precipitate from centrifugation of disrupted biomass.

In another embodiment the food or feed product comprises at least one fraction of Methylotroph bacterial biomass comprises the soluble fraction (SF) as obtainable as the supernatant from centrifugation of disrupted biomass.

In another embodiment the food or feed product comprises at least one fraction of Methylotroph bacterial biomass comprising the acid insoluble fraction (AIF) as obtainable as the insoluble fraction following neutralization of the precipitate from the centrifugation of disrupted biomass.

In another embodiment the food or feed product comprises Methylotroph bacterial biomass from the genus *Methylobacillus.*

In another embodiment the food or feed product comprises Methylotroph bacterial biomass from a *Methylobacillus* strain which does not express exopolysaccharides.

In another embodiment the food or feed product comprises Methylotroph bacterial biomass from *Methylobacillus* which has at least one EPS gene cluster inactivated.

In another embodiment the food or feed product comprises Methylotroph bacterial biomass from *Methylobacillus* which has both EPS gene clusters inactivated.

In another embodiment the food or feed product comprises Methylotroph bacterial biomass wherein said at least one EPS gene cluster or said both EPS gene clusters are entirely or partly deleted (absent).

In another embodiment the food or feed product comprises Methylotroph bacterial biomass which is from *Methylobacillus* which has a decreased, inactivated or deleted polyphosphate kinase (PPK).

In another embodiment the food or feed product comprises Methylotroph bacterial biomass which is from *Methylobacillus* which has a decreased, inactivated or deleted Acyl-homoserine-lactone (AHL) synthase.

In another embodiment the food or feed product comprises Methylotroph bacterial biomass from a bacterium selected from the group consisting of *Methylobacillus flagellatus, Methylobacillus glycogenes, Methylobacillus pratensis, Methylobacillus rhizosphaerae.*

In another embodiment the food or feed product comprises Methylotroph bacterial biomass which is *Methylobacillus flagellatus* biomass.

In another embodiment the food or feed product comprises from 10 %w/w(DM) to 75 %w/w(DM) of said Methylotroph bacterial biomass or a fraction thereof, from 10 %w/w(DM) to 50 %w/w(DM) of said Methylotroph bacterial biomass or a fraction thereof or from 10 %w/w(DM) to 35 %w/w(DM) of said Methylotroph bacterial biomass or a fraction thereof.

In another embodiment the food or feed product comprises Methylotroph bacterial biomass or a fraction thereof in an amount such that its protein contents from said Methylotroph biomass in said food product is in the range from 10 %w/w to 100 %w/w of the total protein contents in said food or feed product, in the range from 10 %w/w to 75 %w/w of the total protein contents in said food or feed product or from 10 %w/w to 50 %w/w of the total protein contents in said food or feed product.

In another embodiment the food or feed product is a solid product.

In another embodiment the food or feed product is a meat like product, such as a meat substitute.

In another embodiment the food or feed product is a feed product.

In another embodiment the food or feed product is an animal feed product for farm animals or for pets.

In another embodiment the food or feed product is a food product.

In another embodiment the food product is a burger patty.

In a further aspect is provided a method for making a food or feed ingredient comprising:
- growing a Methylotroph bacterium to a density allowing the recovery of biomass,
- optionally disrupting said recovered biomass to make a disrupted biomass (DB),
- optionally subjecting said disrupted biomass to centrifugation to produce a pellet-insoluble fraction (IF) and a soluble fraction (SF),
- optionally subjecting the soluble fraction (SF) to acid precipitation followed by neutralization to produce the acid insoluble fraction (AIF),
- reducing the water contents in said biomass, DB, IF, SF or AIF to less than 5.0 %w/w,
to form said food or feed ingredient.

The culture medium employed for growing the Methylotroph bacterium may be any conventional medium suitable for culturing a bacterium cell in question, and may be composed according to the principles of the prior art. The medium will usually contain all nutrients necessary for the growth and survival of the respective bacterium, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GM MY, or Yeast Malt Extract (YM) broth, which are all commercially available. A non-limiting example of suitable media for culturing bacterial cells, such as E. coli cells, including minimal media and rich media such as Luria Broth (LB), M9 media, M17 media, SA media, MOPS media, Terrific Broth, YT and others.

The carbon source may be any suitable carbon substrate known in the art, and in particularly any carbon substrate commonly used in the cultivation of bacteria and/ or fermentation. Non-limiting examples of suitable fermentable carbon substrates include carbohydrates (e.g., C5 sugars such as arabinose or xylose, or C6 sugars such as glucose), glycerol, glycerine, acetate, dihydroxyacetone, one-carbon source, methanol, methane, oils, animal fats, animal oils, plant oils, fatty acids, lipids, phospholipids, glycerolipids, monoglycerides, diglycerides, triglycerides, renewable carbon sources, polypeptides (e.g., a microbial or plant protein or peptide), yeast extract, component from a yeast extract, peptone, casaminoacids or any combination of two or more of the foregoing.

According to some embodiments, the carbon substrate is selected from the group consisting of C5 sugars (such as arabinose or xylose), C6 sugars (such as glucose or fructose), lactose, sucrose, glycerol, glycerine, acetate, Corn steep liquor, yeast extract, component from a yeast extract, peptone, casaminoacids or combinations thereof.

In the present case where the bacterium is a methylotrophic bacterium, the culture medium preferably comprises a reduced one-carbon compound, such as methanol or methylamine, or a multi-carbon compound that contain no carbon-carbon bonds, such as dimethylamine. Thus, according to some embodiments, the culture medium comprises methanol as a carbon source. The concentration of methanol in the culture medium may generally be in the range from about 0,5% w/v to about 4 % w/v, such as from about 2% w/v to about 4 % w/v. According to some embodiments, the concentration of methanol in the culture medium is in the range from about 2,5% w/v to about 3,5% w/v.

As the nitrogen source, various ammonium salts such as ammonia and ammonium sulfate, other nitrogen compounds such as amines, a natural nitrogen source such as peptone, soybeanhydrolysate, and digested fermentative microorganism can be used. As minerals, potassium monophosphate, magnesium sulfate, sodium chloride, ferrous sulfate, manganese sulfate, calcium chloride, and the like can be used.

Suitably, the bacterium is cultivated under suitable conditions for growing the bacterium. Suitable conditions for culturing the respective bacterium are well known to the skilled person. Typically, a bacterium is cultured at a temperature ranging from about 20 to about 45°C, such as from about 30 to about 38°C, such as at about 37°C. The cultivation can be preferably performed under aerobic conditions, such as by a shaking culture, by a stirring culture or in a bioreactor with aeration, at a temperature of about 20 to about 45 °C, such as about 30 to 38 °C, preferably at about 37°C. The pH of the culture is usually above 5, such as in a range from about 6 to about 8, preferably from about 6.5 to about 7.5, more preferably from about 6.8 to about 7.2. The pH of the culture can be adjusted with ammonia, calcium carbonate, various acids, various bases, and buffers. The cultivation may be carried out for a period in the range from 10 to 70 h, preferably in a range from 24 to 60 h, more preferably in a range from 36 to 50 h.

After cultivation, solids such as bacterial biomass can be removed from the culture medium by centrifugation or membrane filtration.

In another embodiment the method comprises the reduction of the water contents of the biomass or fraction thereof by spray drying or by freeze drying.

In another embodiment the method is for making a food ingredient.

In another embodiment the method is for making a feed ingredient.

Typical feeds made from the feed ingredient are e.g. chicken feed or fish feed.

### Examples

### Example 1 - Methylobacillus OCB420 strain and biomass preparation

*Methylobacillus flagellatus* was modified to reduce the viscosity of the broth by deleting the genes responsible for exopolysaccharide formation and cell lysis (WO2022069644A1), generating *Methylobacillus OCB420.* Briefly, a sequence upstream and downstream of the deleted gene were amplified from genomic DNA with PCR. The fragments were appended to an antibiotic resistance cassette, containing its own promoter, terminator and ribosome binding site. The resulting linear construct was transformed into the *Methylobacillus* strain by electroporation. A selection step on antibiotic containing, minimal medium agar plates with methanol as the carbon source was used to identify successful transformants. DNA integration into the correct locus was confirmed by colony PCR. The entire region after integration was amplified from the genome and checked by DNA sequencing.

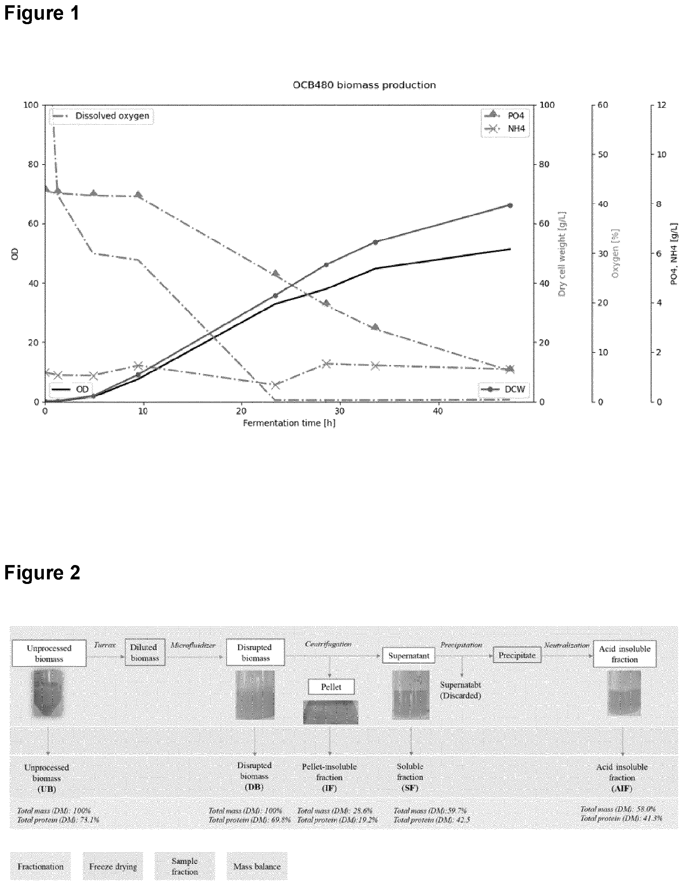

The biomass of *Methylobacillus OCB420* was prepared in 5 L lab-scale bioreactors in fed-batch mode. A mineral medium containing KH2PO4, Na2HPO4, MgSO4, NH4SO4 and a trace element mixture was used. Methanol concentration was dynamically maintained at 4-5 g/L throughout the fermentations via a feedback loop using an online methanol sensor. The pH was kept at 7 by automatic addition of ammonium hydroxide, which also served as source of nitrogen. A bioprocess diagram is depicted in **Figure 1****.** After the culture reached the stationary phase, the biomass was harvested by centrifugation, washed twice with distilled water and stored at -80°C.

### Example 2 - Methylobacillus OCB420 biomass processing

*Methylobacillus OCB420* biomass was freeze-dried to obtain a dry powder termed unprocessed biomass »UB«.

The fractionation process of the biomass was performed in two batches. UB prior to freeze-drying was treated by homogenization, centrifugation, acid precipitation and neutralization. Unprocessed wet biomass was evenly distributed into four centrifugal bottles and Milli-Q water was added to reach a dilution factor of 10. A rotor-stator was used to make a dispersion at a high speed of 10,000 rpm for approximately 30s. To break the bacterial cell wall for the release of protein, the dispersion was further homogenized to produce the disrupted biomass (DB) at 600 bar with a high-pressure homogenizer. DB was further centrifuged at 20,000 g for 30 min. and the soluble supernatant was collected and named as soluble fraction (SF). The insoluble pellet fraction (IF) was collected and freeze-dried for a mass balance calculation and further analysis. The sample was then centrifuged at 10,000 g for 10 min and the supernatant was freeze-dried for a mass balance calculation. The recovered pellet was redispersed into Milli-Q water to create a solution, which was stirred with a magnet at 700 rpm for 1 h and the pH was readjusted to 7 by adding 1 M NaOH. The solution was freeze-dried and named acid-insoluble fraction (AIF).

### Example 3 - effect of freeze drying on Methylobacillus OCB420 biomass

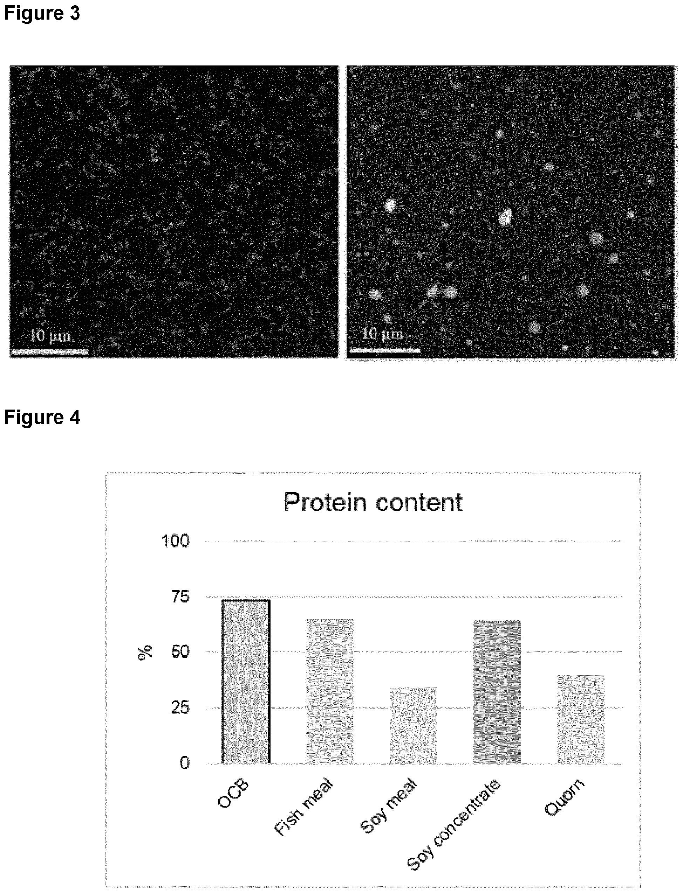

Unprocessed wet biomass was compared to freeze dried biomass to observe the effect of freeze-drying on cell integrity. Samples were stained with Rhodamine-B to stain protein and with Bodipy to stain lipids, then imaged with a confocal laser scanning microscope. The wet biomass is pictured on **Figure 3** **left** and the freeze dried biomass is shown on **Figure 3** **right.** In the freeze-dried biomass the Rhodamine-B signal (protein) is more evenly dispersed, while the Bodipy signal is present in droplets. Few intact cells are visible. This suggests that freeze drying disrupts cell structure to a large degree, dispersing protein content and causing lipids to form droplets.

### Example 4 - Methylobacillus OCB420 biomass compositional analysis

Amino acid compositions of all samples were analysed by standard methods. Cysteine and methionine content was measured using the ISO 13903:2005; EU 152/2009 (F) methods. Tryptophan was measured with the EU 152/2009 method. All other amino acids were measured ISO 13903:2005; EU 152/2009. Total protein content of the samples corresponds to the sum of all amino acid contents. Fat content was measured with the AACC 30-25.01 method. Ash content was determined with the AACC 08-01 method.

To determine nucleotide content, proteins were first precipitated from the samples with 5% Trichloroacetic acid. Nucleic acid-bound proteins were digested by protease, which was inactivated by boiling at the end of digestion. Samples were then treated with nuclease to digest nucleic acids to individual nucleotides, then injected into an HPLC and bases, nucleosides, 5'-NMP and RNA were measured separately.

The results of the analysis are as follows: 73% protein, 2% fat, 5 % ash, 5% nucleotides and 14% other (most likely carbohydrates). The low DNA/RNA content of 5% is notable, comapred to 12-13% in similar bacteria (Volova & Barashkov, 2010). Protein content in the biomass is comparable to other commercial protein fractions, e. g. fish meal (60-72%) or soy protein concentrate (62%), and significantly higher than soy meal (45%) or the popular meat substitute Quorn (40%) (Figure 4).

The composition did not vary much between the different processing methods. The insoluble fraction (IF) had the lowest protein content at 67% and the highest potential carnohydrate content of 27%. The compositions of all sample fractions are summarized in Table 1.

**Table 1. Biomass composition of all sample fractions of Methylobacillus OCB420 biomass.**

| Sample fraction | Total Nitrogen [g/100g] | Protein [g/100g] | Non-Protein Nitrogen [g/100g] | Nitrogen conversion factor (NCF) | Fat [g/100g] | Ash [g/100g] | Total nucleotides [g/100g] | Carbohydrates [g/100g] |
|---|---|---|---|---|---|---|---|---|
| UB | 13.7 ± 0.1 | 73.1 ± 0.3 | 1.6 | 5.35 | 2.0 ± 0.2 | 5.5 ± 0.4 | 5 | 14.4 |
| DB | 13.2 ± 0.3 | 69.8 ± 1.2 | N.A | 5.35 | 1.8 ± 0.01 | 5.4 ± 0.1 | 4.8 | 18.2 |
| SF | 13.4 ± 0.03 | 71.7 ± 0.8 | N.A | 5.35 | 2.1 ± 0.04 | 5.7 ± 0.3 | 5.3 | 15.4 |
| IF | 13.3 ± 0.3 | 67.0 ± 2.4 | 2.2 | 5.17 | 1.2 ± 0.01 | 4.7 ± 0.5 | 3.8 | 23.3 |
| AIF | 14.0 ± 0.2 | 70.9 ± 0.9 | 2.3 | 5.06 | 0.7 ± 0.1 | 5.0 ± 1.3 | 5 | 18.5 |

### Example 5 - Methylobacillus OCB420 protein amino acid ratio

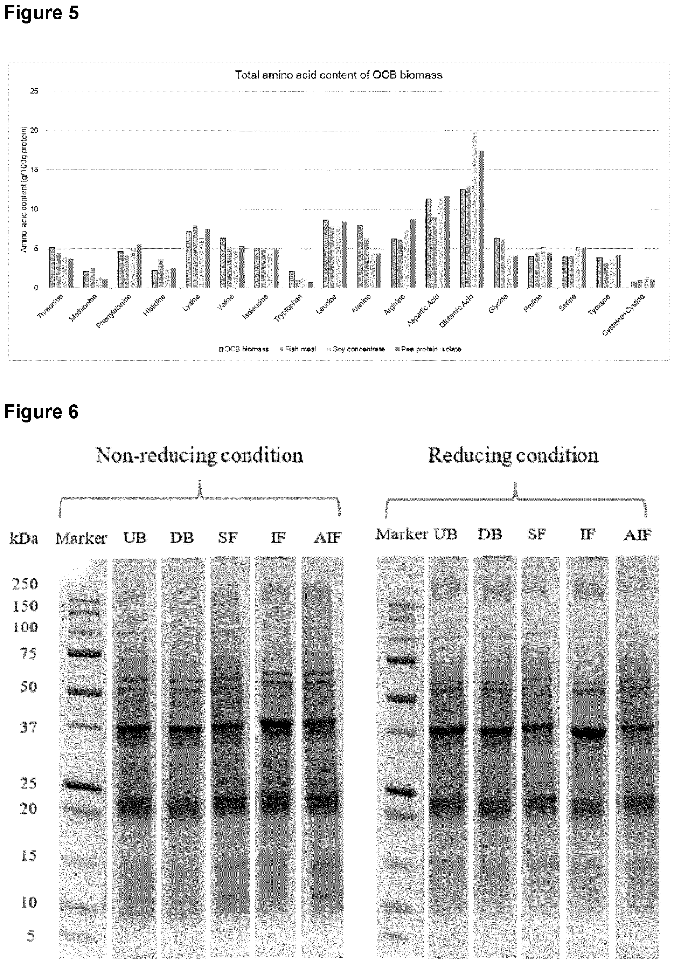

The amino acid content of *Methylobacillus OCB420* biomass was compared to other common protein sources used in food and feed applications, specifically fish meal (Al-Quazzaz, ANIMAL NUTRITION AND FEED TECHNOLOGY 2016, 16(3):527, soy protein concentrate (manufacturer specification) and pea protein concentrate. The results are summarised in Figure 5 and Table 2. Notably, the *Methylobacillus OCB420* biomass has the highest threonine, valine, isoleucine, leucine and tryptophan content of all the protein sources in this comparison. Its methionine and lysine, both of which are important in animal feed, are comparable to fish meal. These findings in combination with the relatively low nucleic acid content make it an interesting alternative to fish meal in feed applications. '

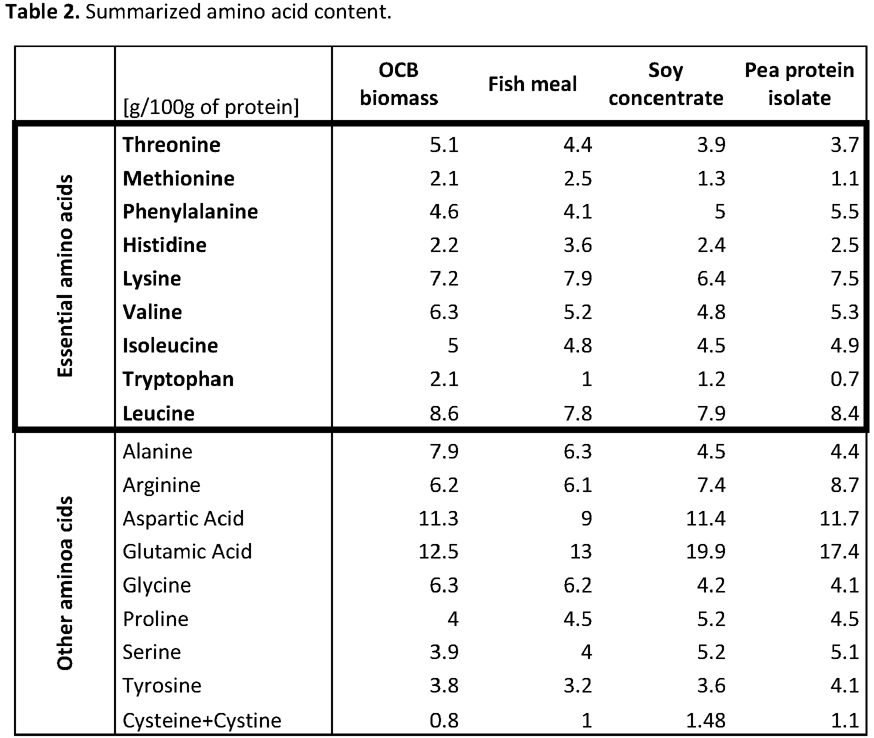

### Example 6 - Methylobacillus OCB420 protein molecular weight profile

The protein molecular weight distribution of the freeze-dried UB, DB, SF, IF and AIF was determined using sodium dodecyl sulphate polyacrylamide gel electrophoresis (SDS-PAGE). Protein solutions were prepared and suspended in sample buffer (0.125M Tris/HCL buffer pH 6.8, 4% w/v SDS, 40% w/v glycerol, 0.02% bromophenol blue). The buffer was diluted in a ratio of 1:1 with 0.9% NaCl-solution for non-reduced samples and β-mercaptoethanol was added in a concentration of 5% for reduced samples. The samples were thoroughly vortexed and heated for 30 min at 100 °C. Afterwards, the samples were centrifuged at 15,000 g for 5 min and 20 µL clear supernatant was loaded onto the gel. The experiment was performed at 200 Volt for 30 min. Subsequently, the gels were stained with Coomassie Blue solution (0.2% CBB R250 in methanol (40%), acetic acid (12%), milliQ-water) overnight. Destaining was also performed overnight using a solution (water, methanol, acetic acid (80/10/10 % v/v)). The protein molecular weight distributions in the soluble biomass fractions UB, DB, SF, IF and AIF are shown in Figure 6. All the samples exhibited similar bands, indicating that none of the fractionation steps affected protein composition. The major bands had a molecular weight of approx. 22, 37, and 55 kDa. Some large bands can be seen between 60 and 150 kDa and small bands with the molecular weight of 10-15 kDa. Further, hardly any differences were found between the sample fractions with or without reducing agent, which indicates the absence of intermolecular disulfide bridges.

### Example 7 - Methylobacillus OCB420 biomass composition and protein secondary structure measured by ATR-FTIR

The protein secondary structure of the freeze-dried UB, DB, SF, IF and AIF was measured using an attenuated-total-reflection (ATR) Fourier Transform Infrared (FTIR) spectrometer with a thermally controlled Bio ATR2 unit at 25 °C and an MCT detector. A 2% w/w solution was prepared by adding the freeze-dried powder in Milli-Q water, followed by vortexing to allow full hydration. Interferograms were accumulated over the spectral range 4000-400 cm-1, with a resolution of 4 cm-1. For evaluation of protein secondary structure, the measured spectra of the amide band I region (1580-1700 cm-1) were vector-normalized using the Bruker OPUS software system. The second derivative was calculated using 9 smoothing points.

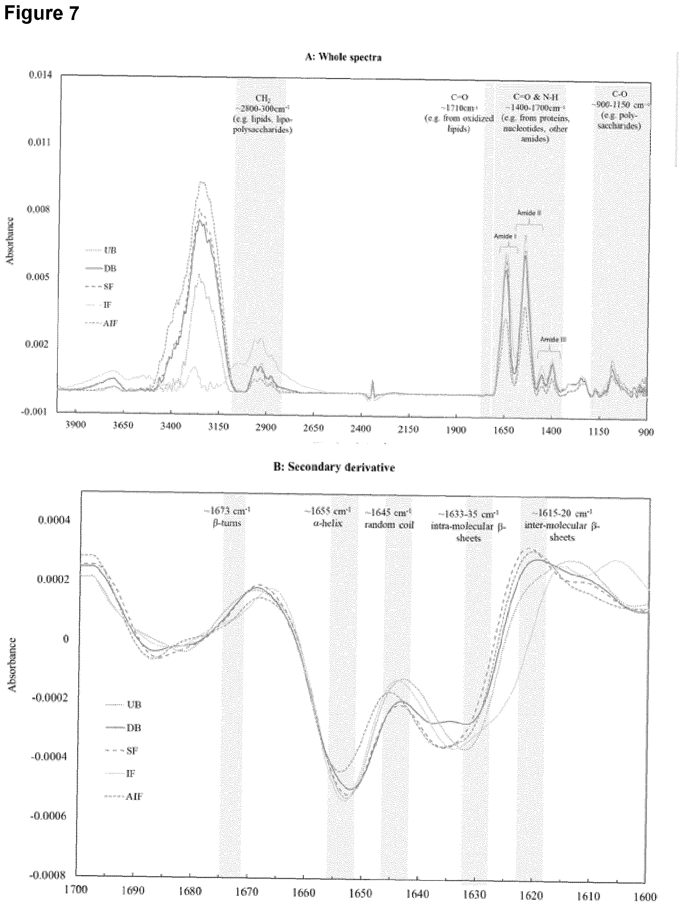

The interaction of infrared light with molecular bonds typical of carbohydrates (C-O bond at 900-1150 cm-1), lipids (CH2 bond at 2800-3000 cm-1), proteins (C-O and NH at 1400-1700 cm-1) and nucleotides (1650 cm-1) gives an indication of the composition of the sample (Figure 7. A). The spectra confirm the composition of the fractions given in **Table 1,** except for the acid precipitated sample (AIF). Another deviation to **Table 1** is the higher CH2 signal indicative for lipids in the pellet fraction (IF) after homogenization treatment and subsequent centrifugation. The high CH2 signal could indicate the presence of other molecules with CH2 signal in that fraction, for example cell wall insoluble lipopolysaccharides, typically for Gram-negative bacteria. Another explanation for the minor compositional deviations of AIF and IF from the overall composition listed in Table 1 can be caused by solubility differences: although no visible precipitation was observed with the measured samples, less soluble molecules can sediment on the ATR surface and are then measured at higher intensity than soluble molecules.

### Example 8 - Thermal stability of Methylobacillus OCB420 biomass

The thermal stability of freeze-dried UB, DB, SF, IF and AIF were analyzed with differential scanning calorimetry (DSC). Freeze dried samples were resuspended of Milli-Q, sealed in a high-volume pan and heated from 25 to 130 °C using a heating rate of 5 °C/min. After 1 min, the pan was cooled down to 25 °C using a cooling rate of 20 °C/min. This heating and cooling process was repeated for a second time to make sure the peak indicated protein denaturation. The onset denaturation temperature (onset Td), the peak temperature of denaturation (Td), and the denaturation enthalpy (J/g biomass) were recorded.

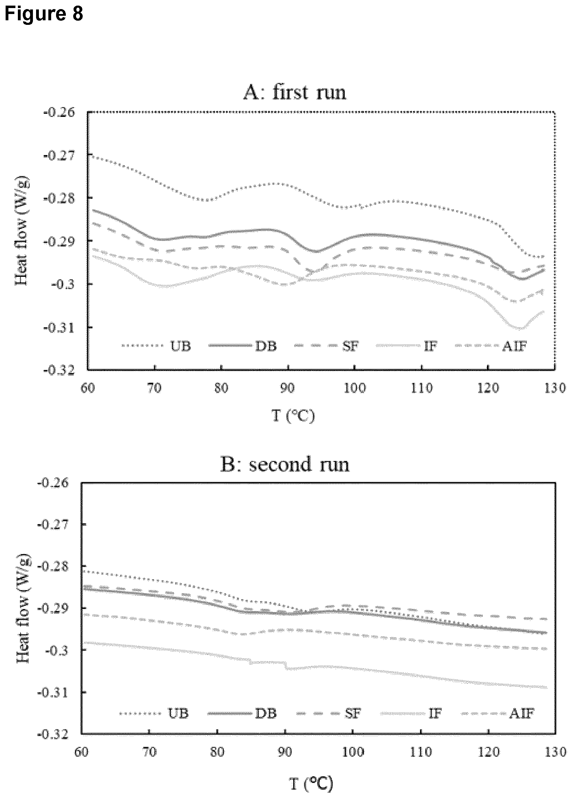

Thermograms of the different biomass fractions were recorded to understand their melting behaviour, an important property for heat induced gel formation. The heat flow change of the different sample fractions UB, DB, SF, IF and AIF is shown in Figure 8 for 2 repeated runs, and the melting temperature (Td) as well as enthalpy are listed in Table 3.

Three different endothermic peaks were observed with all the different fractions during the heating for the first run. The melting temperature (Td) of the first peak was detected between 70 and 76 °C, and 89 to 98 °C for the second peak, and 124 to 126 °C for the third peak. Melting of different cell components may have contributed to these peaks. The denaturation at 95 °C and higher could be linked to the melting of cellular DNA and dehydration/depolymerization of cell wall components. It is noteworthy that the observed enthalpies were mostly far below 0.8 J/g biomaterial which would correspond to approx. less than 1. J/g protein. For comparison, soy protein isolates with a similar protein content gave an enthalpy of 1.3 to 6.9 J/g protein. The decreasing of the melting temperature of samples relative to the UB could be caused by either damaging cell wall components and proteins by microfluidizer treatment or by liberating the proteins from the cell wall material. A second temperature run was performed with each sample and the first and the third peak both disappeared during this second run, indicating that these were irreversibly damaged by the heat treatment during the first run.

**Table 3. The onset of denaturation temperature (onset Td), denaturation peak temperature (Td) and denaturation enthalpy (J/g) with respect to the different fractions of UB, DB, SF, AIF and IF. Values are listed as mean and standard deviation of two independent measurements.**

| Peak | Fractions | UB | DB | SF | IF | AIF |
|---|---|---|---|---|---|---|
| 1^{st} run Peak 1 | Onset *T_{d,1}* ( 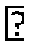 ) | 66.9 ± 0.8 | 65.1 ± 0.8 | 64.8 ± 0.7 | 62.3 ± 1.3 | 72.6 ± 1.1 |
| | *T_{d,1}* ( 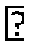 ) | 75.8 ± 0.5 | 70.4 ± 0.1 | 70.8 ± 0.2 | 70.0 ± 1.7 | 75.9 ± 1.1 |
| | Enthalpy (J/g) | 0.5 ± 0.2 | 0.2 ± 0.03 | 0.2 ± 0.01 | 0.8 ± 0.02 | 0.06 ± 0.001 |
| 1^{st} run Peak 2 | Onset *T_{d,2}* ( 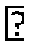 ) | 90.8 ± 0.6 | 89.7 ± 0.01 | 89.9 ± 0.1 | 88.6 ± 0.4 | 83.1 ± 2.4 |
| | *T_{d,1}* ( 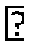 ) | 97.6 ± 0.3 | 94.5 ± 0.4 | 94.3 ± 0.1 | 93.3 ± 0.1 | 89.7 ± 0.9 |
| | Enthalpy (J/g) | 0.3 ± 0.01 | 0.3 ±0.02 | 0.4 ± 0.01 | 0.2± 0.01 | 0.4 ± 0.05 |
| 1^{st} run Peak 3 | Onset *T_{d,3}* ( 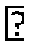 ) | 123.7 ± 1.9 | 121.0 ±0.7 | 118.6 ± 0.1 | 120.0 ± 0.2 | 119.5 ± 0.4 |
| | *T_{d,1}* ( 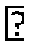 ) | 125.7 ± 0.4 | 124.9 ± 0.01 | 123.6 ± 0.03 | 124.8 ± 0.1 | 124.0 ± 0.4 |
| | Enthalpy (J/g) | 0.1 ± 0.02 | 0.2 ± 0.02 | 0.2 ± 0.01 | 0.3 ± 0.01 | 0.3 ± 0.01 |
| 2^{nd} run Peak 4 | Onset *T_{d,1}* ( 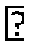 ) | 79.9 ± 0.1 | 78.7 ± 0.9 | 79.9 ± 0.4 | 78.2 | 78.0 ± 0.1 |
| | *T_{d,1}* ( 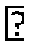 ) | 83.4 ± 0.1 | 83.6 ± 0.2 | 83.9 ± 0.04 | 82.5 | 83.6 ± 0.01 |
| | Enthalpy (J/g) | 0.04 ± 0.01 | 0.1 ± 0.06 | 0.04 ± 0.004 | 0.05 | 0.1 ± 0.05 |
| 2^{nd} run Peak 5 | Onset T*_{d,1}* ( 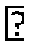 ) | 89.6 ± 0.5 | 89.0 ± 0.7 | 88.4 ± 0.1 | 90.0 | N.D |
| | *T_{d,1}* ( 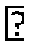 ) | 94.4 ± 0.2 | 92.9 ± 1.2 | 91.9 ± 0.2 | 90.3 | N.D |
| | Enthalpy (J/g) | 0.1 ± 0.01 | 0.2 ± 0.1 | 0.03 ± 0.01 | 0.1 | N.D |

### Example 9 - Zeta potential and conductivity of Methylobacillus OCB420 biomass

The zeta potential of the freeze-dried biomass UB, DB, SF, IF and AIF was measured as described by using a Zetasizer Nano ZS. 2% w/w solutions of the were diluted approximately 100 times. The measurements were performed at 25 °C and the results were expressed as the average from two independent samples. The pH and conductivity were measured after the overnight mixing by a Multi-Parameter Meter.

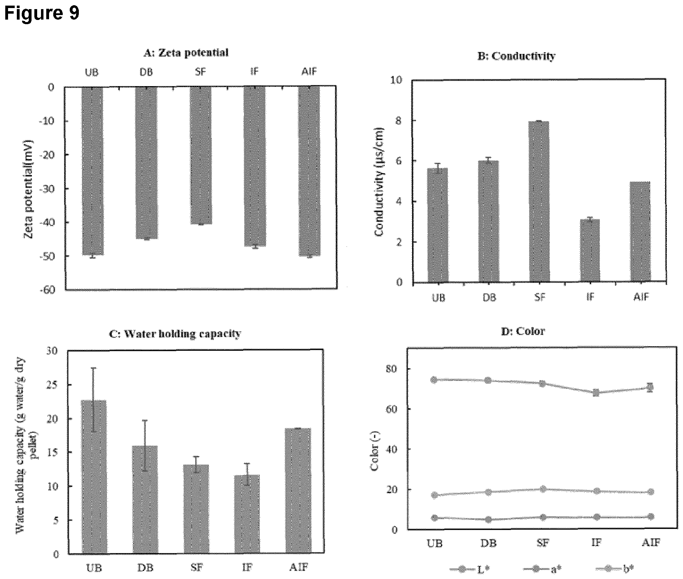

The absolute zeta potential of all samples is very high, ranging between 1501 and 1401 mV (Figure 9 A). It is assumed that the carbohydrates of all samples contributed towards the high negative zeta potential. Although the zeta potential is strongly dependent on the salt content of the sample, the results suggest that the unprocessed biomass fractions were more charged compared with other biomass fractions. The lowest zeta potential (1401 mV) was found for the supernatant (SF). An explanation could be the minor compositional changes of the fractions, as SF had the highest fat content (2%) and at the same time the highest ash content (6%) (including salts as counter ions). The conductivity of the SF sample (8 µS/cm) is significantly higher than for all the other fractions

(Figure 9 B), likewise reflecting a higher salt concentration. For plant protein, the soy protein concentrate (SPC) was reported to have a zeta potential between |20| and |30| at pH 7 and pea protein isolate (PPI) had a zeta potential value between |17| and |25| at pH 7.

### Example 10 - Water holding capacity and color of Methylobacillus OCB420 biomass

To determine the water holding capacity (WHC) of the insoluble matter, 4% w/w dispersions of the freeze-dried fractions UB, DB, SF, IF and AIF in Milli-Q water were vortexed for 1 min and rotated overnight with a roller table. The samples were then centrifuged at 4000 g for 30 min. The supernatant and the pellet were transferred into an aluminum tray and dried in an oven at 105 °C for at least 16 h. The weight of the pellet before (M_{wet pellet}) and after freeze drying (M_{dry pellet}) was measured. The dry masses of the original sample (M_{original}) and of the supernatant (Mₛᵤₚₑᵣₙₐₜₐₙₜ) were measured. The WHC of the dry pellet was calculated according to the formula WHC (g water/g dry biomass) = ((M_{wet pellet})-(M_{dry pellet}))/(M_{dry pellet}). The solubility was calculated according to the formula Solubility (%) = (Mₛᵤₚₑᵣₙₐₜₐₙₜ)/(M_{original}) × 100%.

The water holding capacity (WHC) of the different fractions is shown in Figure 9C. WHC is an important parameter for meat replacement applications, linked to the juiciness of the structure. The UB fraction has the highest WHC of approximately 20 g/g dry pellet. A lower WHC was observed for all the processed biomass of DB, SF and IF. The WHC of all the biomass fractions is in general much higher compared to plant protein sources such as rapeseed, soy and lupine protein (less than 10g /g dry pellet) or microalgae (3.1g/g sample). Specifically, commercial SPC and PPI were reported to have a WHC of approximately 4 g/g sample. Besides, the extraction methods were reported to have a big impact on the WHC, e.g. PPI extracted by alkali-isoelectric precipitation is reported to result in a WHC of 2.4-2.6 g/g sample. Both proteins and polysaccharides can hold water. It is possible that the high WHC is mainly derived from both the protein and residual polysaccharides. WHC is also correlated with mechanical treatment, e.g. higher WHC can be obtained when the porosity of the samples is increased.

### Example 12 - Structuring properties of Methylobacillus OCB420 biomass

The freeze-dried UB and AIF were processed in a high-temperature conical shear cell (HTSC). Heating and cooling were performed using an external oil bath. A protein mixture with the concentration of 40 wt% was prepared by firstly mixing 1% sodium chloride and 59 wt% demineralized water. The sample was mixed with water and hydrated for 30 min. Afterward, the hydrated sample was transferred to the preheated HTSC and sheared for 15 min at 30 rpm at 140.

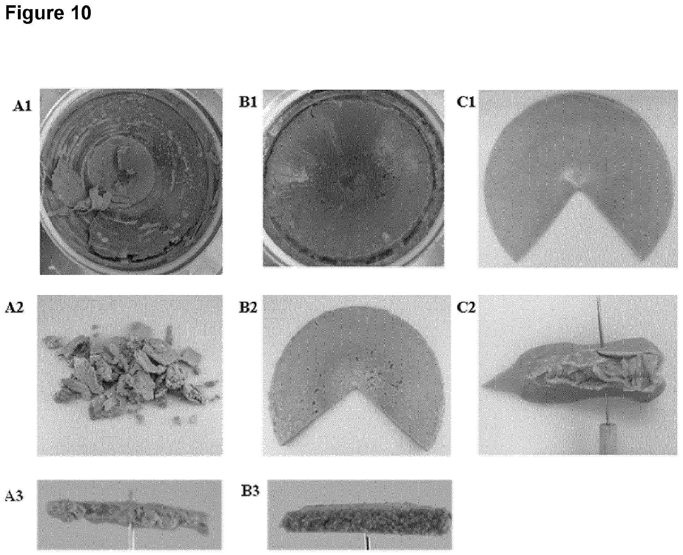

The macrostructure of the sheared and heated UB, AIF, and SPC samples at 40% DM are shown in Figure 10. Both UB and AIF sample formed intact pancakes with an intrinsic smell of cooked meat (umami smell). The UB sample formed a layered structure broken into pieces when taken from the shear cell. The structure resembled minced meat and shows potential for use in meat analogue products. For the AIF sample, a porous pancake like structure was formed. UB might contain a mixture of partly intact cells, contributing towards layer formation by hindering a continuous protein network. For most plant-based meat analogues, either wheat gluten or soy protein are added to achieve the crumbly structure of UB. The use of the unprocessed biomass for meat analogue production could be processed on site of production, saving transport and energy intensive drying steps.

## Claims

1. Use of a protein composition comprising Methylotroph bacterial biomass or at least one fraction thereof for supplementing or substituting proteins in food or feed products.

2. The use according to claim 1, wherein said protein composition comprises Methylotroph bacterial biomass.

3. The use according to claim 1 or 2, wherein said protein composition comprises at least one fraction of Methylotroph bacterial biomass.

4. The use according to any one of claims 1-3, wherein said at least one fraction of Methylotroph bacterial biomass comprises disrupted biomass (DB), the pellet-insoluble fraction (IF) as obtainable as the precipitate from centrifugation of disrupted biomass, the soluble fraction (SF) as obtainable as the supernatant from centrifugation of disrupted biomass, or the acid insoluble fraction (AIF) as obtainable as the insoluble fraction following neutralization of the precipitate from the centrifugation of disrupted biomass.

5. The use according to any one of claims 1-4, wherein said methylotroph bacterial biomass is from the genus *Methylobacillus.*

6. The use according to claim 5, wherein said Methylotroph bacterial biomass is from *Methylobacillus* which does not express exopolysaccharides.

7. The use according to any one of claims 1-6, wherein said Methylotroph bacterial biomass is from *Methylobacillus* which has a decreased, inactivated or deleted polyphosphate kinase (PPK).

8. The use according to any one of claims 1-7, wherein said Methylotroph bacterial biomass is from *Methylobacillus* which has a decreased, inactivated or deleted Acyl-homoserine-lactone (AHL) synthase.

9. The use according to any one of claims 1-8, which comprises the addition of said Methylotroph bacterial biomass or a fraction thereof such that it constitutes from 10%w/w(DM) to 75%w/w(DM) of the food or feed product, from 10%w/w(DM) to 50%w/w(DM) of the food or feed product or from 10%w/w(DM) to 35%w/w(DM) of the food or feed product.

10. Food or feed ingredient comprising Methylotroph bacterial biomass or at least one fraction thereof.

11. The food or feed ingredient according to claim 10, which comprises Methylotroph bacterial biomass.

12. The food or feed ingredient according to claim 10 or 11, which comprises at least one fraction of Methylotroph bacterial biomass, such as disrupted biomass (DB), the pellet-insoluble fraction (IF) as obtainable as the precipitate from centrifugation of disrupted biomass, the soluble fraction (SF) as obtainable as the supernatant from centrifugation of disrupted biomass, or the acid insoluble fraction (AIF) as obtainable as the insoluble fraction following neutralization of the precipitate from the centrifugation of disrupted biomass.

13. Food or feed product comprising Methylotroph bacterial biomass or at least one fraction thereof.

14. The food or feed product according to claim 13, which comprises from 10 %w/w(DM) to 75 %w/w(DM) of said Methylotroph bacterial biomass or a fraction thereof, from 10 %w/w(DM) to 50 %w/w(DM) of said Methylotroph bacterial biomass or a fraction thereof or from 10 %w/w(DM) to 35 %w/w(DM) of said Methylotroph bacterial biomass or a fraction thereof.

15. A method for making a food or feed ingredient comprising:
- growing a Methylotroph bacterium to a density allowing the recovery of biomass,
- optionally disrupting said recovered biomass to make a disrupted biomass (DB),
- optionally subjecting said disrupted biomass to centrifugation to produce a pellet-insoluble fraction (IF) and a soluble fraction (SF),
- optionally subjecting the soluble fraction (SF) to acid precipitation followed by neutralization to produce the acid insoluble fraction (AIF),
- reducing the water contents in said biomass, DB, IF, SF or AIF to less than 5.0 %w/w,
to form said food or feed ingredient.
